(19) Europäisches Patentamt
European Patent Office
Office européen des brevets

(11) Numéro de publication: **0 280 627 B1**

(12) # FASCICULE DE BREVET EUROPEEN

(45) Date de publication de fascicule du brevet: **21.04.93**

(51) Int. Cl.[5]: **C07D 295/22**, C07D 279/00, A61K 31/395

(21) Numéro de dépôt: **88400445.8**

(22) Date de dépôt: **26.02.88**

(54) **Nouveaux dérivés N-substitués de l'alpha-mercaptométhyl benzène propanamide, leur procédé de préparation, leur application à titre de médicaments et les compositions les renfermant.**

(30) Priorité: **26.02.87 FR 8702546**

(43) Date de publication de la demande:
**31.08.88 Bulletin 88/35**

(45) Mention de la délivrance du brevet:
**21.04.93 Bulletin 93/16**

(84) Etats contractants désignés:
**CH DE FR GB IT LI NL**

(56) Documents cités:
**EP-A- 0 115 997**
**EP-A- 0 137 746**

(73) Titulaire: **ROUSSEL-UCLAF**
**35, Boulevard des Invalides**
**F-75007 Paris(FR)**

(72) Inventeur: **Vevert, Jean-Paul**
**55, rue Rouget de l'Isle**
**F-93500 Pantin(FR)**
Inventeur: **Gasc, Jean-Claude**
**6, rue Georges Lyssandre**
**F-93140 Bondy(FR)**
Inventeur: **Delevallée, Françoise**
**48-50, avenue de la Dame Blanche**
**F-94120 Fontenay-Sous-Bois(FR)**
Inventeur: **Petit, Francis**
**119, avenue Carnot**
**F-93140 Bondy(FR)**

(74) Mandataire: **Bourgouin, André et al**
**Département des Brevets ROUSSEL UCLAF**
**111, route de Noisy B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.5x/3.0.1)

**Description**

La présente invention concerne de nouveaux dérivés N-substitués de l'alpha-mercaptométhyl benzène propanamide, leur procédé de préparation, leur application comme médicaments et les compositions les renfermant.

L'invention a pour objet les composés de formule (I) :

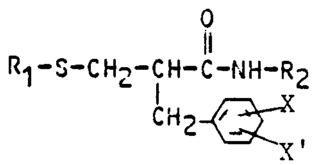

(I)

dans laquelle $R_1$ représente un atome d'hydrogène ou un radical

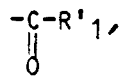

$R'_1$ étant un radical alcoyle renfermant de 1 à 5 atomes de carbone ou un radical aryle, éventuellement substitué par un ou plusieurs radicaux choisis dans le groupe formé par les radicaux hydroxy, alcoyle ou alcoxy renfermant de 1 à 5 atomes de carbone, le radical nitro ou les atomes d'halogène, X et X′, identiques ou différents, représentent un atome d'hydrogène, un radical alcoyle ou alcoxy renfermant de 1 à 5 atomes de carbone, un radical hydroxy, un atome d'halogène ou un radical trifluorométhyle, $R_2$ représente un radical pyrrolidinyle, morpholinyle, pipéridinyle, pipérazinyle, tétrahydrothiazinyle ou hexahydroazépinyle, chacun de ces radicaux étant éventuellement substitué par un ou plusieurs radicaux choisis dans le groupe constitué par les radicaux alcoyle ou alcoxy renfermant de 1 à 5 atomes de carbone, le radical hydroxy, le radical nitro, le radical trifluorométhyle, les radicaux acyles, les atomes d'halogène ainsi que leurs sels d'addition avec les acides.

Parmi les radicaux alcoyle ayant de 1 à 5 atomes de carbone, on peut citer les radicaux méthyle, éthyle, n-propyle, isopropyle, n-butyle, isobutyle, tert-butyle ou n-pentyle.

Parmi les radicaux alcoxy ayant de 1 à 5 atomes de carbone, on peut citer les radicaux méthoxy, éthoxy, n-propoxy, isopropoxy, n-iso ou tertbutoxy.

Les atomes d'halogène peuvent être fluor, chlore, brome, iode.

Lorsque $R_1$ représente un radical

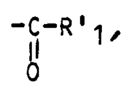

$R'_1$ est de préférence un radical méthyle ou éthyle.

Lorsque $R'_1$ est un radical aryle, il s'agit de préférence d'un radical phényle.

Lorsque $R'_1$ est un radical aryle substitué, il s'agit de préférence d'un radical aryle substitué par un radical choisi dans le groupe formé par le radical hydroxy, les radicaux méthyle et éthyle, les radicaux méthoxy et éthoxy, le radical nitro et l'atome de chlore.

X et X′, identiques ou différents, sont de préférence des atomes d'hydrogène, des radicaux méthyle, éthyle, méthoxy, éthoxy, des atomes de chlore, des radicaux hydroxy ou trifluorométhyle.

Lorsque le radical $R_2$ est substitué, il s'agit de préférence d'un seul substituant, ce substituant est choisi de préférence dans le groupe formé par les radicaux méthyle et éthyle, méthoxy et éthoxy, l'atome de chlore, les radicaux hydroxy, nitro et trifluorométhyle, et plus particulièrement du radical N-acétyle.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcanesulfoniques tels que les acides méthane et éthanesulfoniques, arylsulfoniques, tels que les acides benzène et para-toluènesulfoniques et arylcarboxyliques.

L'invention concerne particulièrement les composés de formule (I) pour lesquels $R_1$ est un atome d'hydrogène, ainsi que leurs sels d'addition avec les acides et ceux pour lesquels $R_1$ est un radical acétyle ainsi que leurs sels d'addition avec les acides.

L'invention a plus particulièrement pour objet les composés de formule (I) pour lesquels $R_2$ est un radical morpholinyle ou pyrrolidinyle ainsi que leurs sels d'addition avec les acides et tout particulièrement l'alphamercaptométhyl N-(4-morpholinyl) benzène propanamide et l'alpha-mercaptométhyl N-(1-pyrrolidinyl) benzène propanamide ainsi que leurs sels d'addition avec les acides.

L'invention a également pour objet un procédé de préparation des produits de formule (I) caractérisé en ce que l'on soumet une forme activée d'un acide de formule (II) :

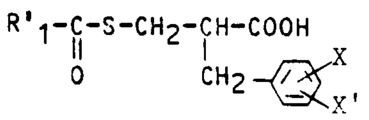

(II)

dans laquelle $R'_1$, X et X' ont les significations indiquées ci-dessus à l'action d'une amine $NH_2$-$R_2$ dans laquelle $R_2$ a la signification déjà indiquée pour obtenir un produit de formule (I) que, le cas échéant, l'on saponifie, pour obtenir un produit de formule (I) dans laquelle $R_1$ représente un atome d'hydrogène, produits de formule (I) que l'on soumet, si désiré, à l'action d'un acide pour en former le sel.

L'activation de la fonction carboxyle du composé de formule (II) pour réaliser la condensation avec l'amine $NH_2$-$R_2$ s'effectue par exemple en présence de dicyclohexylcarbodiimide. On active de préférence l'acide de formule (II) sous la forme d'un chlorure d'acide au sein d'un solvant tel que l'éther, le tétrahydrofuranne, un solvant chloré, notamment le chlorure de méthylène, le 1,1-dichloréthane, le chloroforme ou le tétrachlorure de carbone.

L'invention concerne aussi un procédé de préparation des produits de formule (I) dans laquelle $R_1$, $R_2$, X et X' ont les significations indiquées précédemment, caractérisé en ce que l'on soumet un acide de formule (III) :

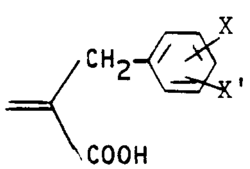

(III)

ou un dérivé fonctionnel de cet acide à l'action d'une amine de formule:

$NH_2$-$R_3$

dans laquelle $R_3$ représente soit $R_2$, $R_2$ étant défini comme ci-dessus, soit $R_2$p, $R_2$p représentant un radical $R_2$ dont les fonctions réactives sont protégées, pour obtenir un produit de formule (IV) :

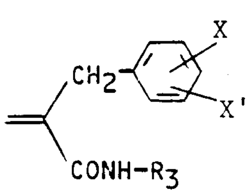

(IV)

dans laquelle X, X' et $R_3$ ont les significations indiquées précédemment que dans le cas où $R_3$ représente $R_2$p, on soumet à un réactif de déprotection pour obtenir un produit de formule (IV') :

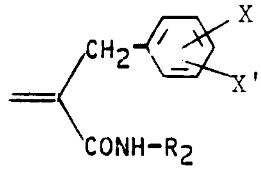

(IV')

dans laquelle X, X' et $R_2$ ont les significations indiquées précédemment puis que l'on soumet à l'action d'un thioacide de formule :

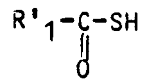

dans laquelle $R'_1$ a la signification précédente pour obtenir un produit de formule (I) dans laquelle $R_1$ représente

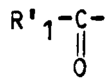

que l'on saponifie, le cas échéant, pour obtenir un produit de formule (I) dans laquelle $R_1$ représente un atome d'hydrogène et produits de formule (I) que, si désiré, l'on salifie par action d'un acide.

Dans des conditions préférentielles de mise en oeuvre du procédé ci-dessus décrit :

- on utilise un dérivé fonctionnel de l'acide de formule (III) tel que le chlorure d'acide, la réaction avec l'amine de formule $NH_2$-$R_3$ est effectuée dans les conditions indiquées précédemment pour la réaction des produits de formule (II) avec l'amine $NH_2$-$R_2$;
- le groupement protecteur des fonctions réactives de $R_2$ est choisi parmi les groupements protecteurs classiques, connus de l'homme de l'art. On peut citer par exemple, les radicaux alcoxycarbonyles, tel que radical terbutoxycarbonyle, les radicaux cycloalcoxycarbonyles, les radicaux aralcoxycarbonyles ;
- la déprotection éventuelle du produit de formule (IV) peut être effectuée par hydrolyse acide, par exemple avec l'acide trifluoroacétique ; on peut cependant utiliser d'autres acides minéraux ou organiques ;
- le thioacide que l'on fait réagir sur le produit de formule (IV') est de préférence l'acide thioacétique ;
- la saponification des produits de formule (I) dans laquelle $R_1$ représente

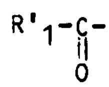

est effectuée par les moyens notamment au moyen d'un hydroxyde de métal alcalin.

Dans certains cas, l'action du thioacide sur le produit de formule (IV') peut également conduire à une substitution au niveau de $R_2$, plus particulièrement sur un atome d'azote. Le produit ainsi obtenu est donc un produit de formule (I) dans laquelle $R_1$ représente

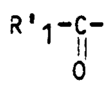

et $R_2$ représente un hétérocycle dont l'atome d'azote est substitué par

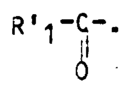

Les composés de formule (I) tels que définis ci-dessus ainsi que leurs sels d'addition avec les acides présentent d'intéressantes propriétés pharmacologiques. Ils présentent, en particulier, de très intéressantes propriétés inhibitrices de l'enképhalinase et sont doués d'une très bonne activité analgésique. Ils présentent aussi des propriétés psychotropes et notamment anti-dépressives et anxiolytiques.

L'enképhalinase est une dipeptidylcarboxypeptidase qui hydrolyse spécifiquement la méthionine et la leucine enképhaline entre le 3ème et le 4ème acide aminé, libérant ainsi un tripeptide Tyr-Gly-Gly (Swerts, J.P., Perdrisot, R., Patey, G., de la Baume, S. and Schwartz, J.C., Europ. J. Pharmacol. (1979), 57, 279).

L'enképhalinase participe ainsi directement à la dégradation physiologique des enképhalinases, ligands naturels endogènes des récepteurs opiacés. Les composés de l'invention, qui retardent la dégradation des enképhalines, stimulent donc les réactions de défense de l'organisme contre la douleur.

Ces propriétés justifient leur application en thérapeutique et l'invention a également pour objet, à titre de médicaments, les produits tels que définis par la formule (I) ci-dessus, ainsi que leurs sels d'addition avec les acides pharmaceutiquement acceptables.

La présente invention a particulièrement pour objet, à titre de médicaments, l'alpha-mercaptométhyl N-(4-morpholinyl) benzène propanamide et l'alpha-mercaptométhyl N-(1-pyrrolidinyl) benzène propanamide ainsi que leurs sels d'addition avec les acides pharmaceutiquement acceptables.

Les médicaments, objet de l'invention, peuvent être utilisés dans le traitement des algies musculaires, articulaires ou nerveuses, des affections rhumatismales, des douleurs dentaires, des zonas et des migraines, ainsi que dans le traitement des maladies inflammatoires, notamment des arthroses, des lumbagos et aussi à titre de traitement complémentaire dans les états infectieux et fébriles. Ils peuvent aussi être utilisés pour traiter les états dépressifs.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 mg et 2 g de principe actif, par jour, par voie orale.

Les produits de départ de formule (II) sont préparés comme indiqué dans le brevet Oudetti et al. US 4 053 651.

Les exmples donnés ci-après illustrent l'invention sans toutefois la limiter.

**Exemple 1 : alpha-[(acétylthio) méthyl] N-(4-morpholinyl) benzène propanamide.**

Une solution renfermant 41,3 g de chlorure d'alpha-(acétylthiométhyl) benzène propanoyle (exemple 1 du B.F. 2 540 495) dans 300 cm3 de chlorure de méthylène est ajoutée goutte à goutte à -10¤C, dans une solution renfermant 33 g de N-aminomorpholine en solution dans 500 cm3 de chlorure de méthylène. Le chlorhydrate d'aminomorpholine précipite. On laisse à température ambiante pendant une nuit, élimine le chlorhydrate d'aminomorpholine et évapore le chlorure de méthylène sous pression réduite. Le solide résiduel est agité pendant 30 minutes dans 300 cm3 d'éther, filtré et séché sous pression réduite. On obtient 44,7 g de produit attendu (86%) qui présente un Rf de 0,23 en chromatographie sur silice (éluant : acétate d'éthyle-chlorure de méthylène 50-50).

**Exemple 2 : alpha-mercaptométhyl N-(4-morpholinyl) benzène propanamide.**

On dissout 2,67 g de produit de l'exemple 1 dans 166 cm3 de méthanol, refroidit à -10¤C et ajoute 82,8 cm3 de soude 0,1N en 45 minutes. On laisse revenir la température à 0¤C en 2 heures, ajoute 82,8 cm3 d'acide chlorhydrique 0,1N en 30 minutes, concentre sous pression réduite à 25¤C, verse le résidu dans un même volume d'eau. On extrait avec du chlorure de méthylène, sèche, filtre, concentre et sèche sous pression réduite. On obtient 2,36 g de produit brut que l'on purifie par chromatographie sur silice

(éluant : acétate d'éthyle 50-chlorure de méthylène 50 MeOH 4) et obtient 1,61 g de produit attendu. F = 110¤C.

**Exemple 3 : alpha-[(acétylthio) méthyl] N-(1-pipéridinyl) benzène propanamide.**

On ajoute à -10¤C, goutte à goutte, une solution de 5,135 g de chlorure d'alpha-(acétylthiométhyl) benzène propanoyle dans 40 cm3 de chlorure de méthylène, dans une solution renfermant 4,01 g de N-aminopipéridine dans 60 cm3 de chlorure de méthylène. Le chlorhydrate d'aminopipéridine précipite. On laisse à température ambiante pendant une nuit, élimine le chlorhydrate d'aminopipéridine et évapore le chlorure de méthylène sous pression réduite. Le solide résiduel est agité 30 minutes dans 50 cm3 d'éther, filtré et séché sous pression réduite. On obtient 6,85 g de produit sous forme d'une huile que l'on purifie par chromatographie sur silice (éluant : chlorure de méthylène-acétate d'éthyle 98-2). On obtient 3,7 g de produit attendu. F = 97¤C.

**Exemple 4 : alpha-mercaptométhyl N-(1-pipéridinyl) benzène propanamide.**

On opère comme à l'exemple 2 à partir de 3,4 g de produit de l'exemple 3, dissout dans 215 cm3 de méthanol, de 106,1 cm3 de soude 0,1N et de 106,1 cm3 d'acide chlorhydrique 0,1N. On obtient 2,56 g de produit attendu après purification par chromatographie sur silice (éluant : acétate d'éthyle-chlorure de méthylène 12-88). F = 119¤C.

**Exemple 5 : alpha [(acétylthio) méthyl] 2,6-dichloro N-(4-morpholinyl) benzène propanamide.**

On refroidit à 0¤C, 17,9 g de chlorure d'acide d'alpha-(acétylthiométhyl) 2,6-dichloro benzène propanoyle en solution dans 400 cm3 de chlorure de méthylène et agite 6,65 cm3 de N-méthylmorpholine puis une solution comprenant 5,9 cm3 d'aminomorpholine dans 200 cm3 de chlorure de méthylène. On agite 18 heures en laissant revenir à température ambiante, lave à l'eau, sèche et élimine les solvants sous pression réduite. Après chromatographie du résidu sur silice (éluant : chlorure de méthylène-méthanol 100-1,5) puis cristallisation dans le mélange éther-chlorure de méthylène 8-2, on recueille 12 g de produit attendu. F = 175¤C.

**Préparation du chlorure d'acide alpha-(acétylthiométhyl) 2,6-dichloro benzène propanoyle utilisé au départ de l'exemple 5.**

**Stade A :** 2,6-dichloro alpha-(diméthylaminométhyl) benzène propanoate de méthyle.

On ajoute sous atmosphère inerte 16 cm3 de diisopropylamine à 250 cm3 de tétrahydrofuranne, refroidit le mélange à -78¤C, ajoute goutte à goutte 65 cm3 de butyllithium, puis la solution de 12,4 g de 3-(diméthylamino) propanoate de méthyle dans 50 cm3 de tétrahydrofuranne. On maintient à -78¤C pendant 30 minutes puis ajoute goutte à goutte 18,1 cm3 d'hexaméthylphosphotriamide. On laisse revenir à température ambiante en agitant pendant 2 heures, ajoute 500 cm3 d'une solution aqueuse saturée en chlorure d'ammonium, puis élimine les solvants sous pression réduite. On extrait la phase aqueuse à l'éther, élimine le solvant sous pression réduite et récupère 28,8 g de produit attendu.

**Stade B :** Iodure de [3-(2,6-dichlorophényl) 2-méthoxycarbonylpropyl] triméthyl ammonium.

On refroidit à 0¤C sous atmosphère inerte 28,8 g de l'ester préparé au stade A en solution dans 140 cm3 d'acétone et ajoute 100 cm3 d'iodure de méthyle. On laisse revenir à température ambiante, agite 2 heures, filtre le précipité, le lave à l'éther et le sèche sous pression réduite. On obtient 53,3 g de produit attendu.

**Stade C :** Acide 2,6-dichloro alpha-méthylène benzène propanoïque.

On ajoute sous atmosphère inerte 120 cm3 de soude 2N à 53,3 g du produit préparé au stade B en suspension dans 500 cm3 de méthanol. On chauffe au reflux pendant 2 heures, élimine les solvants sous pression réduite, extrait la phase aqueuse résiduelle à l'éther, puis après acidification à l'aide d'acide chlorhydrique 5N, on extrait de nouveau à l'éther puis à l'acétate d'éthyle. On réunit les phases organiques, les sèche et élimine les solvants sous pression réduite. On obtient 32 g de produit brut que l'on recristallise

dans l'hexane. F = 132¤C.

**Stade D :** Chlorure d'alpha-[(acétylthio) méthyl] 2,6-dichloro benzène propanoyle.

On ajoute 30 cm3 d'acide thioacétique à 12,7 g du produit préparé au stade C en solution dans 20 cm3 de chlorure de méthylène et agite 16 heures à température ambiante. On élimine le solvant sous pression réduite et obtient 17,2 g de produit attendu.

**Exemple 6 : 2,6-dichloro N-(4-morpholinyl) alpha-mercaptométhyl benzène propanamide.**

On refroidit à 0¤C sous atmosphère inerte 1,7 g de produit obtenu à l'exemple 5 en solution dans 400 cm3 de méthanol et ajoute en 15 minutes 48 cm3 de soude 0,1N. On agite pendant 2 heures à 0¤C, neutralise le milieu réactionnel à l'aide de 5 cm3 d'acide chlorhydrique N, élimine les solvants sous pression réduite à 30¤C. On extrait la phase aqueuse résiduelle au chlorure de méthylène, réunit les phases organiques, les sèche et élimine les solvants sous pression réduite. On récupère, après chromatographie sur silice (éluant : chlorure de méthylène-méthanol 100-4), 1,30 g de produit attendu. F = 142¤C.

**Exemple 7 : alpha-[(acétylthio) méthyl] N-hexahydro 1H-azépinyl) benzène propanamide.**

On opère comme à l'exemple 5 au départ de 2,56 g du chlorure d'alpha(acétylthiométhyl) benzène propanoyle et de 1,26 g de 1-amino homopipéridine. Après chromatographie sur silice (éluant : chlorure de méthylène-méthanol 100-2), on obtient 2 g de produit attendu. F = 126¤C.

**Exemple 8 : N-(hexahydro 1H-azépinyl) alpha-(mercaptométhyl) benzène propanamide.**

On opére comme l'exemple 6 à partir de 2 g du produit préparé comme à l'exemple 7 et 62,8 cm3 de soude 0,1N. Après chromatographie sur silice (éluant : chlorure de méthylène-méthanol 100-2), on obtient 1,7 g de produit attendu. F ≃ 82¤C.

**Exemple 9 : alpha-[(acétylthio) méthyl] N-(1-pyrrolidinyl) benzène propanamide.**

On opère comme à l'exemple 5 au départ de 2,56 g de chlorure d'alpha(acétylthiométhy) benzène propanoyle et de 1,35 g de chlorhydrate de N-aminopyrrolidine et en maintenant la réaction pendant 72 heures. Après chromatographie et trituration du résidu dans l'éther isopropylique, on obtient 0,77 g de produit attendu. F = 91¤C.

**Exemple 10 : alpha-mercaptométhyl N-(1-pyrrolidinyl) benzène propanamide.**

On opère comme à l'exemple 6 à partir de 0,77 g de produit obtenu à l'exemple 9 et 26 cm3 de soude 0,1N. Après chromatographie sur silice (éluant : chlorure de méthylène-méthanol 100-3), on obtient 0,57 g de produit attendu. F = 93¤C.

**Exemple 11 : alpha-[(acétylthio) méthyl] N-(tétrahydro 2H-1,4-thiazin-4-yl) benzène propanamide.**

**Stade A :** 4-[(2-méthylène 3-phényl propanoyl) amino] tétrahydro 2H-1,4-thiazine.

On opère comme à l'exemple 5 au départ de 1,84 g de chlorure d'acide (2-méthylène 3-phényl propanoïque) et 1,2 g de 4-amino tétrahydro 2H-1,4-thiazine. On obtient 1,8 g de produit brut attendu que l'on purifie par chromatographie sur silice (éluant : chlorure de méthylène-méthanol 100-1). F = 141¤C.

**Stade B :** alpha-[(acétylthio) méthyl] N-(tétrahydro 2H-1,4-thiazin-4-yl) benzène propanamide.

On agite pendant 24 heures à température ambiante 0,6 g du produit préparé au stade A dans 10 cm3 d'acide thioacétique. On élimine l'excès d'acide sous atmosphère inerte à 40-45¤C, reprend le résidu dans 200 cm3 de chlorure de méthylène, lave avec une solution de bicarbonate de soude, sèche et élimine le solvant sous pression réduite. On reprend le résidu dans l'éther isopropylique et obtient 0,72 g de produit attendu. F = 138¤C.

**Préparation du 4-amino tétrahydro 2H-1,4-thiazine utilisé au départ de l'exemple 11.**

**Stade A :** 4-nitroso tétrahydro 2H-1,4-thiazine.

On chauffe à 80/85¤C, 16 g de thiomorpholine, 32 cm3 d'acide chlorhydrique dans 120 cm3 d'eau et ajoute en 1 heure une solution de 25 g de nitrite de sodium dans 60 cm3 d'eau. On agite 5 heures à 80¤C, laisse revenir à température ambiante et maintient sous agitation pendant 12 heures. On alcalinise le milieu réactionnel avec une solution aqueuse de potasse 4N et extrait au chlorure de méthylène. On lave la phase organique à l'eau salée, sèche et élimine les solvants sous pression réduite. On récupère 14 g de produit attendu. F ≦ 50¤C.

**Stade B :** 4-amino tétrahydro 2H-1,4-thiazine.

On refroidit à -10¤C, 5,9 g de produit préparé au stade A en solution dans 50 cm3 d'acide chlorhydrique puis ajoute en 1 heure et demie 14,6 g de zinc en poudre. On refroidit à -20¤C pendant 12 heures le milieu réactionnel, le laisse revenir à température ambiante, le verse dans l'eau et ajoute une solution aqueuse saturée en bicarbonate de sodium. On filtre, extrait le filtrat au chlorure de méthylène et à l'acétate d'éthyle, réunit les phases organiques, les sèche et élimine les solvants sous pression réduite. Après chromatographie du résidu sur silice (éluant : chlorure de méthylène-méthanol 100-7), on obtient 2,2 g de produit attendu.

**Exemple 12 : alpha-mercaptométhyl N-(tétrahydro 2H-1,4-thiazin-4-yl) benzène propanamide.**

On opère comme à l'exemple 6 à partir de 0,72 g de produit préparé comme à l'exemple 11 et 22 cm3 de soude 0,1N. Après chromatographie sur silice (éluant : chlorure de méthylène-méthanol 100-2), on obtient 0,56 g de produit attendu. F = 120¤C.

**Exemple 13 : alpha-[(acéthylthio) méthyl] N-[4-acétyl (1-pipérazinyl)] benzène propanamide.**

**Stade A :** 4-[(2-benzyl 1-oxo 2-propényl) amino] 1-pipérazine carboxylate de terbutyle.

On opère comme à l'exemple 5 au départ de 4,54 g de chlorure d'acide (2-méthylène 3-phényl propanoïque) et 3,28 g de 4-amino 1-pipérazine carboxylate de terbutyle en solution dans 100 cm3 de chlorure de méthylène que l'on ajoute à -10¤C en 20 minutes. Après élimination des solvants sous pression réduite, le résidu est repris dans l'éther isopropylique et on obtient après sèchage 7,4 g de produit attendu. F = 160¤C.

**Stade B :** alpha-méthylène N-(1-pipérazinyl) benzène propanamide.

On refroidit à -15¤C sous atmosphère inerte, 7,4 g du produit préparé au stade A dans 20 cm3 de chlorure de méthylène et ajoute goutte à goutte 13 cm3 d'acide trifluoroacétique. On laisse revenir à température ambiante, agite 2 heures, neutralise avec une solution aqueuse saturée en bicarbonate de sodium, extrait à l'acétate d'éthyle, lave à l'eau salée, sèche et élimine les solvants sous pression réduite. On obtient 3,6 g de produit attendu. F = 78¤C.

**Stade C :** alpha-[(acétylthio) méthyl] N-[4-acétyl (1-pipérazinyl)] benzène propanamide.

On agite pendant 72 heures sous atmosphère inerte 1,8 g de produit obtenu comme au stade B dans 20 cm3 d'acide thioacétique. On concentre sous atmosphère inerte, reprend le résidu dans 200 cm3 de chlorure de méthylène, lave avec une solution aqueuse saturée en bicarbonate de sodium, sèche et élimine le solvant sous pression réduite. On chromatographie le résidu sur silice (éluant : chlorure de méthylène-méthanol 100-3) et obtient 2,05 g de produit attendu.

**Le 4-amino 1-pipérazine carboxylate de terbutyle utilisé au départ de l'exemple 13 a été préparé comme suit :**

**Stade A :** Bis-(2-chloroéthyl) carbamate de terbutyle.

On refroidit à 0¤C, 17,2 g de chlorhydrate de bis-chloroéthylamine dans 400 cm3 d'acétonitrile, ajoute 14 cm3 de triéthylamine puis en 20 minutes, 24,5 cm3 de diterbutoxydicarbonate en solution dans 100 cm3 d'acétonitrile. On laisse revenir à température ambiante, agite 16 heures, filtre, concentre le filtrat sous pression réduite, reprend le résidu à l'éther, filtre de nouveau et chasse le solvant. On distille le résidu huileux sous pression réduite ($10^{-1}$mbar) à 80¤C et obtient 18 g de produit attendu.

**Stade B :** 4-amino 1-pipérazine carboxylate de terbutyle.

On mélange sous atmosphère inerte à température ambiante pendant 72 heures, 15,3 g de produit préparé comme au stade A et 80 cm3 d'hydrate d'hydrazine puis ajoute 150 cm3 d'une solution aqueuse saturée en chlorure d'ammonium. On extrait au chlorure de méthylène, lave la phase organique avec une solution aqueuse de chlorure d'ammonium, sèche et élimine le solvant sous pression réduite. Après chromatographie sur silice (éluant : chlorure de méthylène-méthanol 100-7), on obtient 5 g de produit attendu.

**Exemple 14 : N-[4-acétyl (1-pipérazinyl)] alpha-mercaptométhyl benzène propanamide.**

On opère comme à l'exemple 6 au départ de 1,75 g de produit obtenu comme à l'exemple 13 et de 60 cm3 de soude 0,1N. Après chromatographie sur silice (éluant : chlorure de méthylène-méthanol 100-4), on obtient 1,5 g de produit attendu. F __ 50¤C.

| Analyse : $C_{16}H_{23}O_2N_3S$ : 321,426 | | | | |
|---|---|---|---|---|
| Calculé : | C% 59,8 | H% 7,2 | N% 13,1 | S% 10,0 |
| Trouvé : | 60,1 | 7,3 | 13,0 | 9,8 |

**Exemple 15 :**

On a préparé des comprimés répondant à la formule :

| | |
|---|---|
| - Composé de l'exemple 2 | 50 mg |
| - Excipient pour un comprimé terminé à | 350 mg. |
| (Détail de l'excipient : lactose, amidon, stéarate de magnésium, talc). | |

## ETUDE BIOLOGIOUE

### 1) Détermination de l'effet inhibiteur de l'enképhalinase.

L'activité de l'enképhalinase est déterminée à partir d'une fraction membranaire rénale de rat. Les reins sont prélevés sur glace et homogénéisés en tampon HEPES 0.05M pH 7,5 contenant du chlorure de magnésium (50 fois le volume). Après une première centrifugation à 1 500 g, la fraction particulaire est obtenue à l'issue d'une centrifugation à 15 000 g de 20 minutes. Le culot est ensuite lavé et suspendu en tampon HEPES et conservé à -20¤C. Une aliquote de cette préparation membranaire est mise en présence d'octyl béta-D-glucopyranoside (50 mM en centrifugation finale) pendant 15 minutes à 4¤C. Après centrifugation à 100 000 g pendant une heure, le surnageant est prélevé et congelé par aliquotes à -20¤C. La concentration en protéines est déterminée par la méthode au bleu de Comassie.

Une aliquote de la fraction protéinique ainsi préparée est pré-incubée à 25¤C pendant 10 minutes en tampon HEPES 0.05M pH 7,5 ou en présence du produit à étudier. Le substrat ajouté est une enképhaline non naturelle de formule Succinyl-Ala-Ala-Phe-amino-méthylcoumarine (référence 1) (40 µM en concentration finale), l'incubation se poursuit à 37¤C pendant 30 minutes. La réaction est stoppée par chauffage à 95¤C pendant 5 minutes et chaque incubat est centrifugé. Une solution d'aminopeptidase M (4 µM en concentration finale) et de thiorphan (référence 2) ($10^{-5}$M) est ajoutée au surnageant et l'ensemble est porté à 37¤C pendant une heure. La réaction est stoppée comme précédemment décrit et la fluoroescence de la 7-amino-méthyl coumarine ainsi produite déterminée. L'effet des produits à tester est déterminé par le calcul de la $CI_{50}$, concentration qui inhibe de 50% l'hydrolyse du substrat.

Référence 1 : R.A. Munford, P.A. Pierzchala, A-W Strauss and M. Zimmerman. Purification of a membrane-bound metalloendo peptidase from porcine kidney that degrades peptides hormones. Proc. Natl. Acad. Sci. 78, n¤ 11, p. 6623.

Référence 2 : Inhibiteur de l'enképhalinase décrit à l'exemple 20 du B.F. 2 480 747.

Résultats :

| Produit de l'exemple | $CI_{50}$ |
|---|---|
| 2 | $2,2.10^{-7}$M |
| 8 | $3,9.10^{-7}$M |
| 10 | $7,1.10^{-8}$M |
| 12 | $3,5.10^{-7}$M |

**2) Etude de l'activité analgésique chez la souris.**

Etirements provoqués par l'acide acétique chez la souris.

Le test employé est basé sur le fait signalé par R. KOSTER et Coll., (Fed. Proc. (1959), 1B, 412) selon lequel l'injection intrapéritonéale d'acide acétique provoque, chez la souris, des mouvements répétés d'étirement, de torsion pouvant persister pendant plus de 6 heures. Les analgésiques préviennent ou diminuent ce syndrome qui peut être considéré comme l'extériorisation d'une douleur abdominale diffuse. On utilise une solution d'acide acétique à 1% dans l'eau administrée sous un volume de 10 ml/kg.

Le produit étudié est administré par voie buccale une demi-heure avant l'injection d'acide acétique, les souris étant à jeun depuis 6 heures au minimum.

Les étirements sont observés et comptés pour chaque souris pendant une période d'observation de 15 minutes.

Les résultats sont exprimés au moyen de la $DA_{50}$, c'est-à-dire la dose qui permet d'obtenir une diminution de 50% du nombre des étirements par rapport aux animaux témoins. La $DA_{50}$ trouvée est de 80 mg/kg.

**3) Test d'activité antidépressive.**

Les essais sont effectués sur des lots de 5 rats Sprague Dawley. Les animaux, naïfs, sont placés pendant 15 minutes dans un cylindre vertical en plexiglass (diamètre : 18 cm, hauteur : 40 cm) contenant

de l'eau à 25¤C sur une hauteur de 15 cm (épreuve initiale de nage). Ils sont ensuite séchés pendant 15 minutes dans une enceinte chauffée à 32¤C, 24 heures plus tard, ils sont replacés dans le cyclindre rempli d'eau et la durée totale des périodes d'immobilité est mesurée pendant 5 minutes.

Le composé est administré par voir orale successivement 24, 5 et 1 heure avant le test. La première administration a lieu immédiatement après l'épreuve initiale de nage, juste avant de replacer les animaux dans leur boîte d'élevage.

Les moyennes des groupes traités sont comparées à celles du groupe témoin par le test de Dunnett.

Résultats :

| Dose mg/kg produit de l'exemple 2 | Durée d'immobilité en sec (variation %) |
|---|---|
| 0 | 232 ± 7 |
| 20 mg/kg x 3 | 138 ± 20* (-41) |

degré de signification : *$p < 0,05$

**Revendications**

**1.** Les composés de formule (I) :

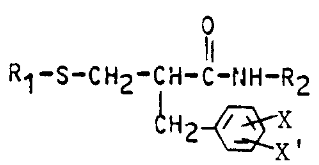

(I)

dans laquelle $R_1$ représente un atome d'hydrogène ou un radical

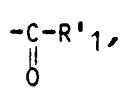

$R'_1$ étant un radical alcoyle renfermant de 1 à 5 atomes de carbone ou un radical aryle, éventuellement substitué par un ou plusieurs radicaux choisis dans le groupe formé par les radicaux hydroxy, alcoyle ou alcoxy renfermant de 1 à 5 atomes de carbone, le radical nitro ou les atomes d'halogène, X et X', identiques ou différents, représentent un atome d'hydrogène, un radical alcoyle ou alcoxy renfermant de 1 à 5 atomes de carbone, un radical hydroxy, un atome d'halogène ou un radical trifluorométhyle, $R_2$ représente un radical pyrrolidinyle, morpholinyle, pipéridinyle, pipérazinyle, tétrahydrothiazinyle ou hexahydroazépinyle, chacun de ces radicaux étant éventuellement substitué par un ou plusieurs radicaux choisis dans le groupe constitué par les radicaux alcoyle ou alcoxy renfermant de 1 à 5 atomes de carbone, le radical hydroxy, le radical nitro, le radical trifluorométhyle, les radicaux acyles,

les atomes d'halogène ainsi que leurs sels d'addition avec les acides.

**2.** Les composés de formule (I) tels que définis à la revendication 1, pour lesquels $R_1$ est un atome d'hydrogène, ainsi que leurs sels d'addition avec les acides.

**3.** Les composés de formule (I) tels que définis à la revendication 1, pour lesquels $R_1$ est un radical acétyle ainsi que leurs sels d'addition avec les acides.

**4.** Les composés de formule (I) tels que définis aux revendications 2 à 3, pour lesquels $R_2$ est un radical morpholinyle ou pyrrolidinyle ainsi que leurs sels d'addition avec les acides.

**5.** Les composés de formule (I) selon la revendication 1 dont les noms suivent :
- l'alpha-mercaptométhyl N-(4-morpholinyl) benzène propanamide, et
- l'alpha-mercaptométhyl N-(1-pyrrolidinyl) benzène propanamide ainsi que leurs sels d'addition avec les acides.

**6.** Procédé de préparation des produits de formule (I), caractérisé en ce que l'on soumet une forme activée d'un acide de formule (II) :

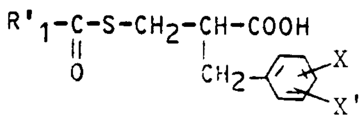

(II)

dans laquelle $R'_1$, X et X′ ont les significations indiquées ci-dessus à l'action d'une amine $NH_2$-$R_2$ dans laquelle $R_2$ a la signification déjà indiquée pour obtenir un produit de formule (I) que, le cas échéant, l'on saponifie, pour obtenir un produit de formule (I) dans laquelle $R_1$ représente un atome d'hydrogène, produits de formule (I) que l'on soumet, si désiré, à l'action d'un acide pour en former le sel.

**7.** Procédé de préparation des produits de formule (I) tels que définis à l'une quelconque des revendications 1 à 4, caractérisé en ce que l'on soumet un acide de formule (III) :

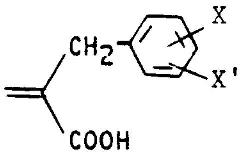

(III)

ou un dérivé fonctionnel de cet acide à l'action d'une amine de formule :

$NH_2$-$R_3$

dans laquelle $R_3$ représente soit $R_2$, $R_2$ étant défini comme ci-dessus, soit $R_2p$, $R_2p$ représentant un radical $R_2$ dont les fonctions réactives sont protégées, pour obtenir un produit de formule (IV) :

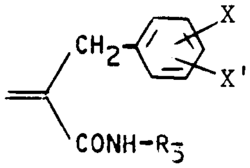

(IV)

dans laquelle X, X′ et $R_3$ ont les significations indiquées précédemment que dans le cas où $R_3$ représente $R_2p$, on soumet à un réactif de déprotection pour obtenir un produit de formule (IV′) :

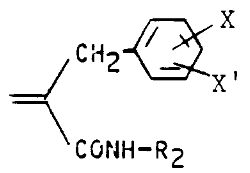

(IV')

dans laquelle X, X′ et $R_2$ ont les significations indiquées précédemment puis que l'on soumet à l'action d'un thioacide de formule :

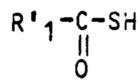

dans laquelle $R'_1$ a la signification précédente pour obtenir un produit de formule (I) dans laquelle $R_1$ représente

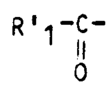

que l'on saponifie, le cas échéant, pour obtenir un produit de formule (I) dans laquelle $R_1$ représente un atome d'hydrogène et produits de formule (I) que, si désiré, l'on salifie par action d'un acide.

**8.** A titre de médicaments, les produits de formule (I) tels que définis à l'une quelconque des revendications 1 à 4, ainsi que leurs sels d'addition avec les acides pharmaceutiquement acceptables.

**9.** A titre de médicaments, les produits tels que définis à la revendication 5, ainsi que leurs sels d'addition avec les acides pharmaceutiquement acceptables.

**10.** Les compositions pharmaceutiques contenant à titre de principe actif, l'un au moins des médicaments tels que définis par l'une des revendications 8 ou 9.

## Claims

**1.** The compounds of formula (I):

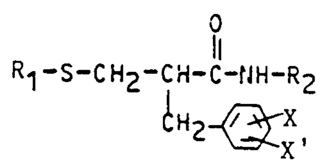

(I)

in which $R_1$ represents a hydrogen atom or a

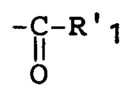

radical, $R'_1$ being an alkyl radical containing 1 to 5 carbon atoms or an aryl radical, optionally substituted by one or more radicals chosen from the group formed by hydroxy radicals, alkyl or alkoxy radicals containing 1 to 5 carbon atoms, the nitro radical or halogen atoms, X and X', identical or different, represent a hydrogen atom, an alkyl or alkoxy radical containing 1 to 5 carbon atoms, a hydroxy radical, a halogen atom or a trifluoromethyl radical, $R_2$ represents a pyrrolidinyl, morpholinyl,

piperidinyl, piperazinyl, tetrahydrothiazinyl or hexahydroazepinyl radical, each of these radicals being optionally substituted by one or more radicals chosen from the group constituted by alkyl or alkoxy radicals containing 1 to 5 carbon atoms, the hydroxy radical, the nitro radical, the trifluoromethyl radical, acyl radicals, halogen atoms, as well as their addition salts with acids.

**2.** The compounds of formula (I) as defined in claim 1, for which $R_1$ is a hydrogen atom, as well as their addition salts with acids.

**3.** The compounds of formula (I) as defined in claim 1, for which $R_1$ is an acetyl radical, as well as their addition salts with acids.

**4.** The compounds of formula (I) as defined in claims 2 to 3, for which $R_2$ is a morpholinyl or pyrrolidinyl radical, as well as their addition salts with acids.

**5.** The compounds of formula (I) according to claim 1 of which the names follow:
- alpha-mercaptomethyl N-(4-morpholinyl) benzene propanamide, and
- alpha-mercaptomethyl N-(1-pyrrolidinyl) benzene propanamide, as well as their addition salts with acids.

**6.** Preparation process for the products of formula (I), characterized in that an activated form of an acid of formula (II):

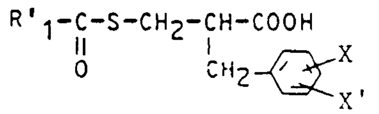

(II)

in which $R'_1$, X and X' have the meanings indicated above, is subjected to the action of an amine $NH_2$-$R_2$ in which $R_2$ has the meaning already indicated, in order to obtain a product of formula (I) which, if appropriate, is saponified, in order to obtain a product of formula (I) in which $R_1$ represents a hydrogen atom, which products of formula (I) are subjected, if desired, to the action of an acid in order to form the salt.

**7.** Preparation process for the products of formula (I) as defined in any one of claims 1 to 4, characterized in that an acid of formula (III):

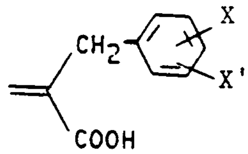

(III)

or a functional derivative of this acid, is subjected to the action of an amine of formula:

$NH_2$-$R_3$

in which $R_3$ represents either $R_2$, $R_2$ being defined as above, or $R_2p$, $R_2p$ representing an $R_2$ radical the reactive functions of which are protected, in order to obtain a product of formula (IV):

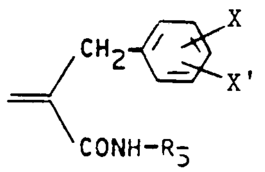

(IV)

in which X, X' and $R_3$ have the meanings indicated previously, which when $R_3$ represents $R_2p$, is subjected to a deprotection reagent in order to obtain a product of formula (IV'):

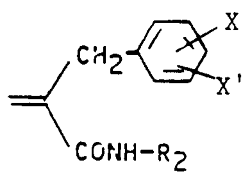

(IV')

in which X, X' and $R_2$ have the meanings indicated previously, which is then subjected to the action of a thioacid of formula:

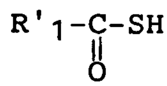

in which $R'_1$ has the previous meaning, in order to obtain a product of formula (I) in which $R_1$ represents

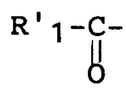

which is saponified, if appropriate, in order to obtain a product of formula (I) in which $R_1$ represents a hydrogen atom, and which products of formula (I) are, if desired, salified by the action of an acid.

**8.** As medicaments, the products of formula (I) as defined in any one of claims 1 to 4, as well as their addition salts with pharmaceutically acceptable acids.

**9.** As medicaments, the products as defined in claim 5, as well as their addition salts with pharmaceutically acceptable acids.

**10.** The pharmaceutical compositions containing as active ingredient at least one of the medicaments as defined in one of claims 8 or 9.

**Patentansprüche**

**1.** Verbindungen der Formel (I)

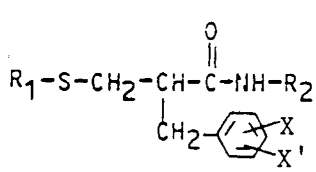

(I)

worin $R_1$ ein Wasserstoffatom oder einen Rest

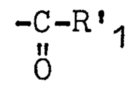

darstellt, in dem $R'_1$ einen Alkylrest mit 1 bis 5 Kohlenstoffatomen oder einen Arylrest bedeutet, welcher gegebenenfalls substituiert ist durch einen oder mehrere Reste, ausgewählt unter den Hydroxy-, Alkyl- oder Alkoxyresten mit 1 bis 5 Kohlenstoffatomen, der Nitrogruppe oder den Halogenatomen, X und X', die identisch oder voneinander verschieden sind, ein Wasserstoffatom, einen Alkyl- oder Alkoxyrest mit 1 bis 5 Kohlenstoffatomen, eine Hydroxygruppe, ein Halogenatom oder eine Trifluormethylgruppe wiedergeben, $R_2$ für einen Pyrrolidinyl-, Morpholinyl-, Piperidinyl-, Piperazinyl-, Tetrahydrothiazinyl- oder Hexahydroazepinyl-Rest steht, wobei ein jeder dieser Reste gegebenenfalls substituiert ist durch einen oder mehrere Reste, ausgewählt unter den Alkyl- oder Alkoxyresten mit 1 bis 5 Kohlenstoffatomen, der Hydroxygruppe, der Nitrogruppe, der Trifluormethylgruppe, den Acylresten und den Halogenatomen, sowie deren Additionssalze mit Säuren.

**2.** Verbindungen der Formel (I) gemäß Anspruch 1, worin $R_1$ für ein Wasserstoffatom steht, sowie deren Additionssalze mit Säuren.

**3.** Verbindungen der Formel (I) gemäß Anspruch 1, worin $R_1$ einen Acetylrest darstellt, sowie deren Additionssalze mit Säuren.

**4.** Verbindungen der Formel (I) gemäß den Ansprüchen 2 und 3, worin $R_2$ einen Morpholinyl- oder Pyrrolidinylrest bedeutet, sowie deren Additionssalze mit Säuren.

**5.** Verbindungen der Formel (I) gemäß Anspruch 1 mit den folgenden Bezeichnungen:
α-Mercaptomethyl-N-(4-morpholinyl)-benzolpropanamid und
α-Mercaptomethyl-N-(1-pyrrolidinyl)-benzolpropanamid
sowie deren Additionssalze mit Säuren.

**6.** Verfahren zur Herstellung der Produkte der Formel (I), dadurch gekennzeichnet, daß man eine aktivierte Form einer Säure der Formel (II)

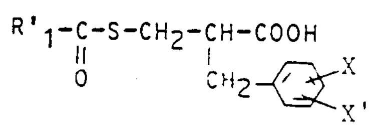

(II)

worin $R'_1$, X und X' die vorstehend angegebene Bedeutung besitzen, der Einwirkung eines Amins $NH_2$-$R_2$, worin $R_2$ die angegebene Bedeutung besitzt, unterzieht, um zu einem Produkt der Formel (I) zu gelangen, welches man gegebenenfalls verseift, um ein Produkt der Formel (I) zu erhalten, worin $R_1$ für ein Wasserstoffatom steht, und die Produkte der Formel (I) gewünschtenfalls der Einwirkung einer Säure unterzieht, um hieraus das Salz zu bilden.

**7.** Verfahren zur Herstellung der Produkte der Formel (I), wie in einem der Ansprüche 1 bis 4 definiert, dadurch gekennzeichnet, daß man eine Säure der Formel (III)

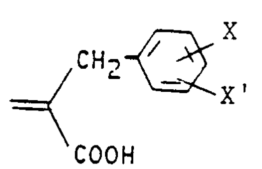

(III)

oder ein funktionelles Derivat dieser Säure der Einwirkung eines eins der Formel

$NH_2$-$R_3$

unterzieht, worin $R_3$ entweder $R_2$ bedeutet, wobei $R_2$ wie vorstehend definiert ist, oder $R_2p$ bedeutet, wobei $R_2p$ einen Rest $R_2$ wiedergibt, dessen reaktive Funktionen geschützt sind, um zu einem Produkt der Formel (IV)

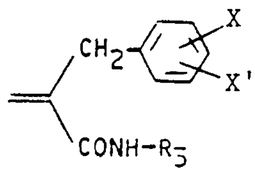

(IV)

zu gelangen, worin X, X' und $R_3$ die vorstehend angegebenen Bedeutungen besitzen, in dem Fall, in dem $R_3$ für $R_2p$ steht, man einem Reagens zur Schutzgruppenabspaltung unterzieht, um zu einem Produkt der Formel (IV')

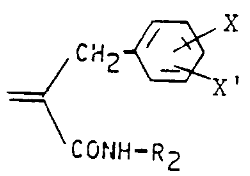

(IV')

zu gelangen, worin X, X' und $R_2$ die vorstehend angegebenen Bedeutungen besitzen, wonach man der Einwirkung einer Thiosäure der Formel

$$R'_1-\underset{\underset{O}{\|}}{C}-SH$$

unterzieht, worin $R'_1$ die vorstehende Bedeutung besitzt, um zu einem Produkt der Formel (I) zu gelangen, worin $R_1$ für

$$R'_1-\underset{\underset{O}{\|}}{C}-$$

steht, welches man gegebenenfalls verseift, um zu einem Produkt der Formel (I) zu gelangen, worin $R_1$ für ein Wasserstoffatom steht, und gewünschtenfalls die Produkte der Formel (I) durch Einwirken einer Säure in ein Salz überführt.

**8.** Als Arzneimittel die Produkte der Formel (I), wie in einem der Ansprüche 1 bis 4 definiert, sowie deren Additionssalze mit pharmazeutisch verträglichen Säuren.

**9.** Als Arzneimittel die Produkte, wie in Anspruch 5 definiert, sowie deren Additionssalze mit pharmazeutisch verträglichen Säuren.

**10.** Pharmazeutische Zusammensetzungen, enthaltend als Wirkstoff zumindest eines der Arzneimittel, wie in einem der Ansprüche 8 oder 9 definiert.